# EUROPÄISCHE PATENTANMELDUNG

(11) **EP 4 763 832 A1**
(43) Veröffentlichungstag der Anmeldung: **24.06.2026**
(21) Anmeldenummer: 24222374.1
(22) Anmeldetag: 20.12.2024
(51) Int. Cl.: C07C 273/18, C07C 275/30, C07C 275/64

(54) **VERFAHREN ZUR HERSTELLUNG VON SUBSTITUIERTEN PHENYLHARNSTOFFDERIVATEN**

(71) Anmelder: LANXESS Deutschland GmbH, 50569 Köln (DE)
(72) Erfinder: BÖGER, Uwe, 51375 Leverkusen (DE); MÜLLER, Mauritio, 83022 Rosenheim (DE); LEIBERICH, Ricarda, 63150 Heusenstamm (DE); HALLE, Olaf, 51061 Köln (DE); SUBERG, Marcus, 41352 Korschenbroich (DE); PRINZ, Thomas, 51371 Leverkusen (DE)

(57) **Zusammenfassung**

Die vorliegende Erfindung betrifft ein neues Verfahren zur Aufreinigung von substituierten Phenylharnstoffderivaten, insbesondere von 3-(3,4-Dichlorphenyl)-1,1-dimethylharnstoff.

## Beschreibung

Die vorliegende Erfindung betrifft ein neues Verfahren zur Herstellung von substituierten Phenylharnstoffderivaten, insbesondere von 3-(3,4-Dichlorphenyl)-1,1-dimethylharnstoff.

### Stand der Technik

Phenylharnstoffe sind eine Klasse von selektiv wirksamen Herbiziden; so ist 3-(3,4-Dichlorphenyl)-1,1-dimethylharnstoff (DCMU), das die Photosynthese von Pflanzen hemmt. Es wird zur völligen Beseitigung von Pflanzen verwendet (Breitbandherbizid) ebenso wie zum Schutz von Holz und Mauerwerk und als Beschichtungsmittel.

Im Stand der Technik sind verschiedene Ansätze zur Herstellung von Phenylharnstoffderivaten bekannt, insbesondere die Herstellung von 3-(3,4-Dichlorphenyl)-1,1-dimethylharnstoff (DCMU) aus 3,4-Dichlorphenylisocyanat und Dimethylamin, die entweder in Lösungsmitteln oder in der Schmelze durchgeführt werden.

Bei den in DE-A-2206167 und DD-A-201140 beschriebenen Schmelzeverfahren, die als Semi-Batch-Prozesse in gerührten Reaktoren durchgeführt werden, muss aufgrund der hohen Exothermie der Reaktion durch geeignete technische Maßnahmen eine ausreichende Wärmeabfuhr und damit Temperaturkontrolle sichergestellt werden. Hierfür werden beide Edukte dosierkontrolliert zugegeben, was durch die damit verbundenen langen Verweilzeiten bei Temperaturen oberhalb des Schmelzpunkts des Produkts zur Bildung unerwünschter Nebenprodukte führt. Bei durch unzureichende Durchmischung hervorgerufenem lokalen Aminmangel wird das Isocyanat nicht hinreichend schnell umgesetzt, was zur thermischen Zersetzung oder unerwünschter Dimerisierung und Trimerisierung (Tris-(3,4-dichlorphenyl)-1,3,5-triazin-2,4,6-trion) des Isocyanats führt.

Aus EP23210489.3 ist ein Schmelzeverfahren zur Herstellung von substituierten Phenylharnstoffderivaten bekannt, bei dem die Umsetzung von substituierten Phenylharnstoffderivaten mit Dimethylamin und/oder N,O-Dimethylhydroxylamin in einem Reaktor durchgeführt wird, der ein Verhältnis von Oberfläche zu Volumen von 100 bis 5000m²/m³ aufweist.

Die vorgenannten Reaktionsbedingungen führen zwar dazu, dass die substituierten Phenylharnstoffderivate in hoher Ausbeute erhalten werden, jedoch ist es wünschenswert mit einem zeiteffizienten und technisch einfachen Verfahren die Reinheit des Produktes weiter zu erhöhen.

### Aufgabe der vorliegenden Erfindung

Die Aufgabe der vorliegenden Erfindung war es daher, ein effizientes Verfahren zur Herstellung von substituierten Phenylharnstoffderivaten, insbesondere von 3-(3,4-Dichlorphenyl)-1,1-dimethylharnstoff, in hoher Reinheit bereitzustellen, welches zeiteffizient und technisch einfach durchgeführt werden kann.

### Lösung der Aufgabe

Überraschenderweise wurde nun gefunden, dass die der Erfindung zugrunde liegende Aufgabe dadurch gelöst wird, dass die Herstellung von substituierten Phenylharnstoffderivaten, insbesondere von 3-(3,4-Dichlorphenyl)-1,1-dimethylharnstoff, in einem Reaktor in einem Reaktor mit einem Verhältnis von Oberfläche zu Volumen von 100 bis 5000 m²/m³ bei Temperaturen, die 1 bis 30°C über dem Schmelzpunkt des substituierten Phenylharnstoffderivates der Formel (I) liegen und bei einem Druck von 5 bis 100 bar, welches sich dadurch kennzeichnet, dass die Reaktionsmischung nach der Umsetzung mittels einer Düse zerstäubt und auf einen Druck < 5 bar entspannt wird, wobei die Temperatur nach der Zerstäubung unterhalb des Schmelzpunktes des substituierten Phenylharnstoffderivates liegt.

### Gegenstand der Erfindung

Gegenstand der Erfindung ist somit ein Verfahren zur Herstellung von substituierten Phenylharnstoffderivaten der Formel (I) 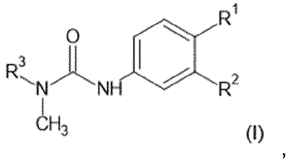
mit R¹ = H, Methyl, F, Cl oder Br,
R² = H, F, CI, Br, vorzugsweise Cl, oder C₁ - C₃-Alkyl, oder einfach oder mehrfach mit F, Cl oder Br substituiertes C₁ - C₃-Alkyl, vorzugsweise CF₃,
wobei R¹ und R² nicht gleichzeitig H bedeuten
   und
R³ = CH₃ oder OCH₃,
die durch die Umsetzung von Verbindungen der Formel (II) 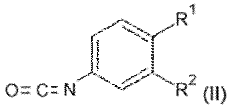
mit einem Amin der Formel (III) 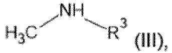
wobei R¹, R² und R³ die vorgenannte Bedeutung haben,
in einem Reaktor mit einem Verhältnis von Oberfläche zu Volumen von 100 bis 5000 m²/m³ bei Temperaturen, die 1 bis 30°C über dem Schmelzpunkt des substituierten Phenylharnstoffderivates der Formel (I) liegen und bei einem Druck von 5 bis 100 bar, wobei die Reaktionsmischung nach der Umsetzung mittels einer Düse zerstäubt und auf einen Druck von 1 bis < 5 bar, vorzugsweise 1 bar, entspannt wird, und wobei die Temperatur nach der Zerstäubung unterhalb des Schmelzpunktes des substituierten Phenylharnstoffderivates der Formel (I) liegt.

Als Düse im Sinne der Erfindung wird vorzugsweise ein beliebig geformtes, vorzugsweise ein rohrförmiges Bauteil verstanden, welches eine Querschnittsverengung aufweist und Flüssigkeiten zerstäubt. Als Düse sind Einstoffdüsen, wie Hohlkegeldüsen, Hohlkegel-Druckdüsen, oder Zweistoffdüsen und/oder pneumatische Zerstäuber bevorzugt.

In einer weiteren bevorzugten Ausführungsform der Erfindung ist die Düse beheizt auf Temperaturen von 1 bis 30°C über dem Schmelzpunkt des substituierten Phenylharnstoffderivates der Formel (I).

Der Düsendurchmesser liegt vorzugsweise im Bereich von 0,05 mm bis 1mm, vorzugsweise 0,1 mm- 0,25mm. Der Durchsatz in den Düsen liegt vorzugsweise bei von 0,3 l/h bis 2,4 l/h. Ebenfalls bevorzugt ist eine Druckdifferenz von Reaktorausgang zum Düsenaustritt von 5 bis 60 bar.

Das Zerstäuben im Sinne der Erfindung erfolgt vorzugsweise von der Reaktionsmischung in Form einer Schmelze in Tropfen.

Vorzugsweise führt die Absenkung der Temperatur zum Auskristallisieren des substituierten Phenylharnstoffderivates der Formel (I).

Besonders bevorzugt handelt es sich bei den substituierten Phenylharnstoffderivaten der Formel (I) um die nachstehend genannten Verbindungen: 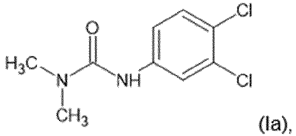 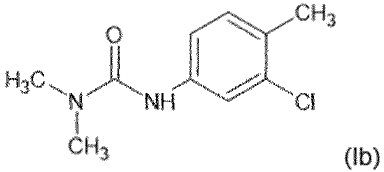 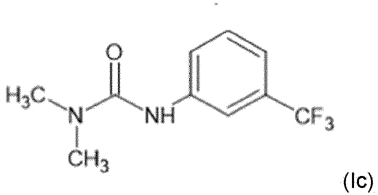 oder

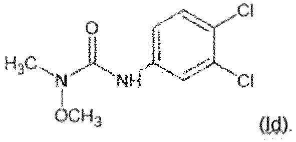

Die Verbindung der Formel (la) 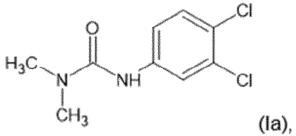 wird vorzugsweise hergestellt über die Umsetzung von 3,4-Dichlorphenylisocyanat mit Dimethylamin.

Die Verbindung der Formel (Ib) 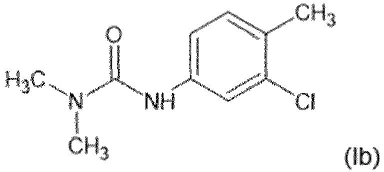 wird dabei vorzugsweise hergestellt über die Umsetzung von 3-Chlor-4-methylphenylisocyanat mit Dimethylamin.

Die Verbindung der Formel (Ic) 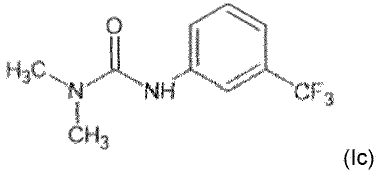 wird dabei vorzugsweise hergestellt über die Umsetzung von 3-Trifluormethylphenylisocyanat mit Dimethylamin.

Die Verbindung der Formel (Id) 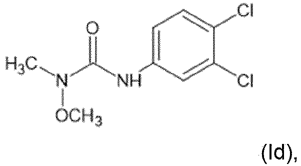 wird dabei vorzugsweise hergestellt über die Umsetzung von 3,4-Dichlorphenylisocyanat mit einem Amin der Formel (III)

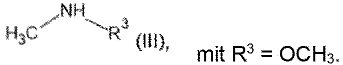

In einer Ausführungsform der Erfindung wird die Umsetzung einem Reaktor durchgeführt bei Temperaturen, die 1 bis 30°C über dem Schmelzpunkt des substituierten Phenylharnstoffderivates der Formel (I) liegen.

In einer bevorzugten Ausführungsform der Erfindung beträgt das Verhältnis von Oberfläche zu Volumen im Reaktor 500 bis 3500m²/m³, besonders bevorzugt 650 bis 2000m²/m³.

Unter Reaktor im Sinne der Erfindung wird vorzugsweise ein rohrförmiger Reaktor verstanden. Bevorzugt sind Rohrreaktoren, besonders bevorzugt Rohrbündelreaktoren, oder Mikroreaktoren. Der bevorzugte rohrförmige Reaktor wird vorzugsweise von einem Kühlmedium, wie beispielsweise Thermalöl, umflossen.

In einer besonders bevorzugten Ausführungsform der Erfindung weisen der Reaktor bzw. bei einem Rohrbündelreaktor die einzelnen Rohre des Reaktors einen Durchmesser von 0,8 mm bis 40 mm, besonders bevorzugt 2 bis 6 mm auf. Die Reaktorlänge beträgt vorzugsweise 0,4 bis 3 m.

In einer weiteren bevorzugten Ausführungsform der Erfindung sind die Reaktoren mit statischen Mischern ausgestattet.

Ein statischer Mischer oder Statikmischer ist eine Vorrichtung zum Mischen von Fluiden, in der vorzugsweise allein die Strömungsbewegung die Vermischung bewirkt und der nicht über bewegte Elemente verfügt. Er besteht vorzugsweise aus strömungsbeeinflussenden Elementen in einem Rohr, bevorzugt aus einem oder mehreren unterschiedlich angeordneten Elementen, die den Stoffstrom abwechselnd teilen und wieder zusammenführen, wodurch die Vermischung erreicht wird.

In einer weiteren bevorzugten Ausführungsform der Erfindung beträgt die Verweilzeit im Reaktor 5-120 Sekunden, vorzugsweise 20-60 Sekunden.

Des Weiteren ist bevorzugt, dass der Anteil von Wasser und/oder organischen Lösungsmitteln, wie vorzugsweise Toluol, 0 - 0,2 Gew.%, bezogen auf die Gesamtmischung, beträgt.

Zudem ist es bevorzugt, dass die Herstellung kontinuierlich durchgeführt wird, d.h. beide Edukte kontinuierlich im gewünschten molaren Verhältnis dem Reaktor zugeführt werden.

In einer bevorzugten Ausführungsform der Erfindung beträgt das molare Verhältnis von Amin der Formel (III) zum substituierten Phenylisocyanat der Formel (II) 2 : 1 bis 10 : 1, vorzugsweise 3 : 1 bis 6 : 1. In dem erfindungsgemäßen Verfahren wird das substituierte Phenylisocyanat vorzugsweise über Düsen in das vorzugsweise im Überschuss vorliegende Amin der Formel (III) eingeleitet.

In einer weiteren bevorzugten Ausführungsform der Erfindung erfolgt das erfindungsgemäße Verfahren bei einem Druck von 5 bis 60 bar, besonders bevorzugt 10 bis 55 bar.

In einer weiteren bevorzugten Ausführungsform der Erfindung wird das erfindungsgemäße Verfahren bei einer Temperatur von 1 bis 30°C über dem Schmelzpunkt des substituierten Phenylharnstoffderivates der Formel (I), vorzugsweise bei einem Druck von 1 bis 100 bar, durchgeführt, im Falle von 3-(3,4-Dichlorphenyl)-1,1-dimethylharnstoff also bei 160 bis 190°C, vorzugsweise bei einem Druck von 1 bis 100 bar.

Das bei dem erfindungsgemäßen Verfahren vorzugsweise im molaren Überschuss eingesetzte Amin der Formel (III) wird nach der Umsetzung mit dem substituierten Phenylisocyanat der Formel (II), vorzugsweise dem 3,4-Dichlorphenylisocyanat, vom Endprodukt, dem substituierten Phenylharnstoffderivat der Formel (I), abgetrennt und das überschüssige Amin vorzugsweise wieder in den Reaktor zurückgeführt. Dies kann beispielsweise durch Rückführung in den Aminvorratsbehälter erfolgen aus dem dann die Zudosierung in den Reaktor erfolgt.

In dem erfindungsgemäßen Verfahren erfolgt am Ende des Reaktionsrohres die Entspannung auf einen Druck 1 bis < 5 bar und die Zerstäubung der Reaktionsmischung, vorzugsweise der Schmelze mittels einer Düse, vorzugsweise in Tropfen.

In einer Ausführungsform der Erfindung ist die Düse beheizt auf Temperaturen von 1 bis 30°C über dem Schmelzpunkt des substituierten Phenylharnstoffderivates der Formel (I). Die Entspannung erfolgt dabei in einen Behälter, in dem die Temperatur nach der Zerstäubung unterhalb des Schmelzpunktes des substituierten Phenylharnstoffderivates liegt.

Das Produkt fällt vorzugsweise unmittelbar nach der Düse als weißer fein pulvriger Feststoff an.

### Bevorzugte Ausführungsform des Verfahrens:

In einer bevorzugten Ausführungsform der Erfindung erfolgt das erfindungsgemäße Verfahren am Beispiel der Herstellung von 3-(3,4-Dichlorphenyl)-1,1-dimethylharnstoff wie folgt:
In einen öltemperierten Rohrreaktor mit einem Verhältnis von Oberfläche zu Volumen von 1000m²/m³ und mit einem Durchmesser von 4 mm, ausgestattet mit statischen Mischern, wird auf 120 bis 150°C vortemperiertes Dimethylamin eingeleitet. Über eine am Anfang des Rohrreaktors eingebaute Düse wird auf 100-120°C vorgeheiztes 3,4-Dichlorphenylisocyanat so eingeleitet, dass ein molares Verhältnis von Dimethylamin zu 3,4-Dichlorphenylisocyanat von 3 : 1 bis 10: 1 eingehalten wird. Die Vermischung von Dimethylamin mit 3,4-Dichlorphenylisocyanat erfolgt idealerweise mit einem statischen Mischer. Der Druck liegt vorzugsweise bei 20 - 50 bar, die Reaktortemperatur bei 160 bis 185°C und die Verweilzeit des Reaktionsmediums bei ca. 30s. Am Ende des Reaktionsrohres erfolgte eine Entspannung der Reaktionsmischung, vorzugsweise einer Schmelze vorzugsweise über ein beheiztes Ventil mit nachgeschalteter beheizter Düse in einen gekühlten konischen Sprühturm, wobei vorzugsweise das im Überschuss eingesetzte Amin als Gasphase abgetrennt wird. Das Produkt fällt unmittelbar nach der Düse als weißer fein pulvriger Feststoff an, der in einem angeschlossenen Auffangbehälter gesammelt wird.

Für die anderen substituierten Phenylharnstoffderivate gemäß Formel (I) erfolgt das erfindungsgemäße Verfahren analog. Die Reaktortemperaturen werden entsprechend des Schmelzpunkts des substituierten Phenylharnstoffderivates der Formel (I) angepasst und liegen vorzugsweise bei einer Temperatur von 1 bis 30°C über dem Schmelzpunkt des substituierten Phenylharnstoffderivates der Formel (I).

Im Folgenden wird die Erfindung an Beispielen und Vergleichsbeispielen beschrieben. Die folgenden Beispiele sind jedoch nur bevorzugte Beispiele für die vorliegende Erfindung und die vorliegende Erfindung ist nicht auf die folgenden Beispiele beschränkt.

### Beispiele

### Beispiel 1 (Vergleichsbeispiel) gemäß EP23210489.3:

Zur kontinuierlichen Herstellung von 3-(3,4-Dichlorphenyl)-1,1-dimethylharnstoff (Diuron) wurden In ein öltemperiertes Reaktionsrohr mit einem Innendurchmesser von 4mm , ausgestattet mit statischen Mischern, wurden 262g/h auf 120°C vortemperiertes Dimethylamin eingeleitet. Über eine unmittelbar am Anfang des Reaktionsrohrs eigebaute Düse wurden 273g/h (molares Verhältnis DMA/3,4-DCPI: 4) auf 130°C vorgeheiztes 3,4-Dichlorphenylisocyanat in das Dimethylamin eingedüst und mithilfe der statischen Mischer zügig vermischt. Der Systemdruck lag bei 53bar, die Öltemperatur bei 163°C, die Verweilzeit des Reaktionsmediums bei ca. 60s.

Am Ende des Reaktionsrohres erfolgte eine Entspannung auf Umgebungsdruck über ein beheiztes Ventil in einen Entspannungsbehälter. Das schmelzförmige Reaktionsprodukt wurde danach in dem auf 165°C beheizten Entspannungsbehälter vom im Überschuss eingesetzten Amin befreit. Die Verweilzeit im Behälter lag bei 30 min- 1 h.

Es zeigten sich nach Abkühlen der Reaktionsmischung (Schmelze) deutliche bräunliche Verfärbungen beim Produkt, das noch einen merklichen Amingeruch aufwies. Die Abgasleitungen der Anlage hinter dem Entspannungsbehälter wiesen ein merkliches Fouling auf, was teilweise zu Verstopfungen führte.

Der Umsatz lag bei >99,9% bezogen auf das eingesetzte Isocyanat, die Reinheit des gewonnenen Produkts bei >99,7%. Kritische Nebenprodukte wie 3,4,3',4'-Tetrachlorazobenzol und 3,4,3',4'-Tetrachlorazoxybenzol waren nicht nachweisbar.

### Beispiel 2 (erfindungsgemäß):

Zur kontinuierlichen Herstellung von 3-(3,4-Dichlorphenyl)-1,1-dimethylharnstoff (Diuron) wurden In ein öltemperiertes Reaktionsrohr mit einem Innendurchmesser von 4mm, ausgestattet mit statischen Mischern, wurden 262g/h auf 120°C vortemperiertes Dimethylamin eingeleitet. Über eine unmittelbar am Anfang des Reaktionsrohrs eigebaute Düse wurden 273g/h (molares Verhältnis DMA/3,4-DCPI: 4) auf 130°C vorgeheiztes 3,4-Dichlorphenylisocyanat in das Dimethylamin eingedüst und mithilfe der statischen Mischer zügig vermischt. Der Systemdruck lag bei 53 bar, die Öltemperatur bei 163°C, die Verweilzeit des Reaktionsmediums bei ca. 60s.

Nach der Umsetzung wurde die Reaktionsmischung (Schmelze) am Ende des Reaktionsrohres über ein beheiztes Ventil mit nachgeschalteter beheizter Düse (Düsendurchmesser von 0,1 mm bei einem Durchsatz von 600 ml/h und einer Druckdifferenz von 52 bar) in einen gekühlten konischen Sprühturm überführt und auf einen Druck von 1 bar entspannt, wobei die Temperatur nach der Zerstäubung bei 20°C - 40°C, vorzugsweise bei 30°C, lag. Bei der Entspannung entwich überschüssiges Amin als Gas. Das Produkt fiel unmittelbar nach der Düse als weißer fein pulvriger Feststoff an, der in einem angeschlossenen Auffangbehälter gesammelt wurde. Die Verweilzeit im Sammelbehälter lag bei 30 min- 1 h. Das erhaltene Produkt war weiß, und zeigt keinen Amingeruch mehr. Die Abgasleitungen der Anlage hinter dem Sprühturm wiesen kein mehr Fouling auf.

Der Umsatz lag bei >99,9% bezogen auf das eingesetzte Isocyanat, die Reinheit des gewonnenen Produkts bei >99,7%. Kritische Nebenprodukte wie 3,4,3',4'-Tetrachlorazobenzol und 3,4,3',4'-Tetrachlorazoxybenzol waren nicht nachweisbar.

## Patentansprüche

1. Verfahren zur Herstellung von substituierten Phenylharnstoffderivaten der Formel (I) 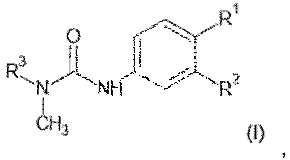
mit R¹ = H, Methyl, F, Cl oder Br,
R² = H, F, Cl, Br, vorzugsweise Cl, oder C₁ - C₃-Alkyl, oder einfach oder mehrfach mit F, CI oder Br substituiertes C₁ - C₃-Alkyl, vorzugsweise CF₃,
wobei R¹ und R² nicht gleichzeitig H bedeuten
und
R³ = CH₃ oder OCH₃,
durch die Umsetzung von Verbindungen der Formel (II) 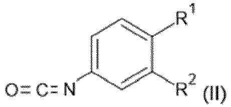
mit einem Amin der Formel (III) 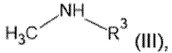
wobei R¹, R² und R³ die vorgenannte Bedeutung haben,
in einem Reaktor mit einem Verhältnis von Oberfläche zu Volumen von 100 bis 5000 m²/m³ bei Temperaturen, die 1 bis 30°C über dem Schmelzpunkt des substituierten Phenylharnstoffderivates der Formel (I) liegen und bei einem Druck von 5 bis 100 bar, **dadurch gekennzeichnet, dass** die Reaktionsmischung nach der Umsetzung mittels einer Düse zerstäubt und auf einen Druck 1 bis < 5 bar entspannt wird, wobei die Temperatur nach der Zerstäubung unterhalb des Schmelzpunktes des substituierten Phenylharnstoffderivates der Formel (I) liegt.

2. Verfahren nach Anspruch 1, **dadurch gekennzeichnet, dass** es sich bei den Verbindungen der Formel (I) um 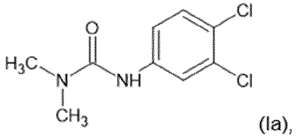 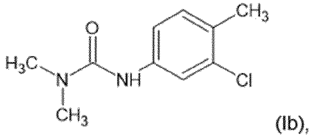 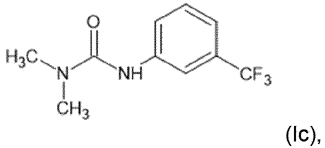 oder 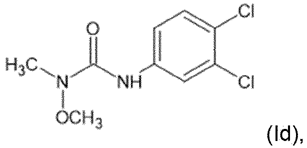 handelt.

3. Verfahren nach Anspruch 1 bis 2, **dadurch gekennzeichnet, dass** es sich bei dem Reaktor um einen Rohrreaktor handelt.

4. Verfahren nach wenigstens einem der Ansprüche 1 bis 3, **dadurch gekennzeichnet, dass** der Rohrreaktor einen Durchmesser von 0,8 mm bis 40 mm aufweist.

5. Verfahren nach wenigstens einem der Ansprüche 1 bis 4, **dadurch gekennzeichnet, dass** es sich bei dem Rohrreaktor um einen Rohrbündelrektor oder einen Mikroreaktor handelt.

6. Verfahren nach wenigstens einem der Ansprüche 1 bis 5, **dadurch gekennzeichnet, dass** die Reaktion kontinuierlich durchgeführt wird.

7. Verfahren nach wenigstens einem der Ansprüche 1 bis 6, **dadurch gekennzeichnet, dass** das Amin nach Formel (III) und das substituierte Phenylisocyanat nach Formel (II) im molaren Verhältnis von 2 : 1 bis 10:1, vorzugsweise 3: 1 bis 6: 1, eingesetzt werden.

8. Verfahren nach wenigstens einem der Ansprüche 1 bis 7, **dadurch gekennzeichnet, dass** die Umsetzung bei einem Druck von 5 bis 100 bar, vorzugsweise 5 bis 60 bar, besonders bevorzugt 10 bis 55 bar durchgeführt wird.

9. Verfahren nach wenigstens einem der Ansprüche 1 bis 8, **dadurch gekennzeichnet, dass** es sich bei der Düse um ein rohrförmiges Bauteil handelt, welches eine Querschnittsverengung aufweist und Flüssigkeiten zerstäubt.

10. Verfahren nach Ansprüche 9, **dadurch gekennzeichnet, dass** es sich bei der Düse um Einstoffdüsen vorzugsweise Hohlkegeldüsen oder Hohlkegel-Druckdüsen, oder um Zweistoffdüsen und/oder pneumatische Zerstäuber handelt.

11. Verfahren nach wenigstens einem der Ansprüche 1 bis 9, **dadurch gekennzeichnet, dass** die Verweilzeit im Reaktor bei 5 bis 120 Sekunden beträgt.
